# FASCICULE DE BREVET EUROPEEN

(11) **EP 1 251 121 B1**
(45) Date de publication et mention de la délivrance du brevet: **01.10.2003**
(21) Numéro de dépôt: 02290872.7
(22) Date de dépôt: 08.04.2002
(51) Int. Cl.: C07C 275/14, C07C 273/02, A61K 7/48, A61K 7/42, A61K 7/02, C09B 1/26

(54) **Dérivés anthraquinoniques, procédé de fabrication, utilisation comme pigments et compositions cosmétiques**
Anthraquinonderivate, Verfahren zu ihrer Herstellung und ihre Anwendung als Pigmente sowie Kosmetische Zubereitungen
Anthraquinone derivatives, process for their preparation and use as pigments and cosmetic compositions

(30) Priorité: 11.04.2001 FR 0104989
(43) Date de publication de la demande: 23.10.2002
(73) Titulaire: L'OREAL, 75008 Paris (FR)
(72) Inventeur: Bordier, Thierry, 93290 Tremblay (FR); Philippe, Michel, 91320 Wissous (FR)
(74) Mandataire: Casalonga, Axel

(56) Documents cités:
- DE-C- 212 436
- FR-A- 2 039 381
- FR-A- 2 125 026
- FR-A- 2 330 742
- US-A- 5 486 629

## Description

L'invention est relative à une nouvelle famille de composés anthraquinoniques, à leur procédé de fabrication et à leur utilisation comme pigments, notamment en cosmétique. L'invention vise également des compositions cosmétiques les contenant.

Dans la littérature, la synthèse de colorants à noyau anthraquinonique, en particulier ceux substitués par des aminés, est bien connue. Une synthèse est notamment décrite dans le brevet FR-A-2 039 381. Certains de ces colorants, tels que ceux décrits dans le brevet US-A-5 486 629, sont utilisés dans la teinture des cheveux.

Ces colorants sont en général solubles dans l'eau ou dans des milieux apolaires (solvants organiques, huiles). Ils ne peuvent donc pas être utilisés comme pigments insolubles dans des applications comme le maquillage de la peau, un phénomène de relargage étant observé.

Par conséquent, cette famille de colorants possède un très faible potentiel de développement industriel comme pigment même si certains procédés de polymérisation, comme celui décrit dans le brevet US-A-4 279 662, ou de traitement complexe comme celui décrit dans le brevet FR-A-2 060 730, ont tenté d'améliorer ce problème persistant.

La Demanderesse vient de découvrir qu'une nouvelle famille de composés anthraquinoniques à jonction urée pouvait être utilisée comme pigment, en particulier dans des compositions cosmétiques. Ces composés, objet de l'invention, sont insolubles ou très peu solubles à la fois dans les milieux aqueux, les solvants organiques et les milieux huileux. Ils permettent donc de résoudre le problème de relargage observé avec les colorants anthraquinoniques de l'art antérieur, dans ces milieux, et en particulier dans les milieux huileux.

En outre, ces nouveaux composés sont très accessibles car ils sont préparés en une seule étape dont la mise en oeuvre est aisée.

La présente invention a ainsi pour objet des nouveaux composés anthraquinoniques de formule (I) ci-dessous.

Un autre objet de l'invention est un procédé de fabrication de ces composés nouveaux.

L'invention a encore pour objet l'utilisation comme pigment de ces composés, notamment en cosmétique.

L'invention a également pour objet des compositions cosmétiques les contenant.

D'autres objets, caractéristiques, aspects et avantages de l'invention apparaîtront encore plus clairement à la lecture de la description et des divers exemples qui suivent.

Selon l'invention, les composés nouveaux répondent à la formule générale (I) : 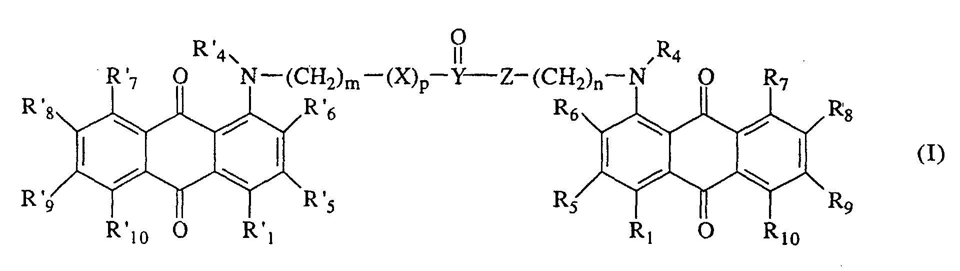 dans laquelle :
m et n, identiques ou différents, sont compris entre 1 et 20 ;
X et Z représentent chacun NH ou O ;
p vaut 0 ou 1 ;
Y représente un atome de carbone ou un atome de soufre ;
R₁, R'₁, R₅, R'₅, R₆, R'₆, R₇, R'₇, R₈, R'₈, R₉, R'₉, R₁₀, R'₁₀, identiques ou différents, représentent chacun un atome d'hydrogène, un atome d'halogène, un groupe hydrocarboné en C₁₋₈, linéaire ou ramifié, saturé ou insaturé, un groupe hydroxy,
   ―O―R₁₁, 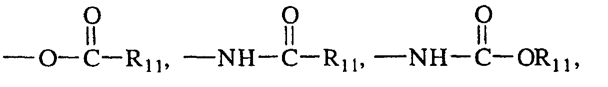 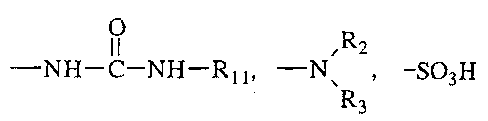 ou ses sels, 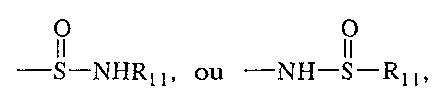
R₂, R₃, R₄, R'₄, et R₁₁, identiques ou différents, représentent chacun un atome d'hydrogène, un groupe hydrocarboné en C₁₋₈, linéaire ou ramifié, saturé ou insaturé, éventuellement hydroxylé ;

Les groupes hydrocarbonés convenant dans la présente invention sont linéaires ou ramifiés, saturés ou insaturés, et comportent de 1 à 8 atomes de carbone, de préférence de 1 à 4 atomes de carbone.

A titre de groupe hydrocarboné, on peut notamment citer les groupes alkyle, alcényle ou alcynyle, comme, par exemple, les groupes méthyle, éthyle, n-propyle, iso-propyle, n-butyle, iso-butyle, tert-butyle, méthylène, éthényle, ou éthynyle.

Comme atome d'halogène, on peut notamment citer les atomes de chlore, de brome, de fluor et d'iode, de préférence l'atome de chlore.

Les composés préférés de l'invention sont ceux répondant à la formule (I) dans laquelle n=m, R'₁=R₁, R'₄=R₄, R'₅=R₅, R'₆=R₆, R₇'=R₇, R₈'=R₈, R₉'=R₉ et R₁₀'=R₁₀, Z=X et p=1, et les composés plus particulièrement préférés répondent à la formule générale (II) suivante : 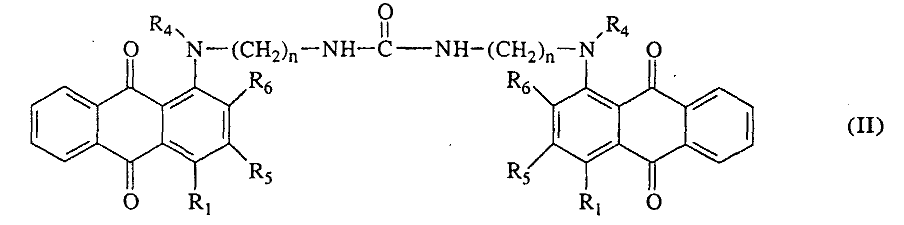 dans laquelle :
n vaut de 1 à 12,
R₁ représente un atome d'hydrogène, un atome d'halogène, un groupe hydrocarboné en C₁-C₈, linéaire ou ramifié, saturé ou insaturé, un groupe hydroxy, ou un groupe -NR₂R₃,
R₂, R₃ et R₄ représentent chacun un atome d'hydrogène ou un groupe hydrocarboné en C₁-C₈, linéaire ou ramifié, saturé ou insaturé,
R₅ et R₆ représentent chacun un atome d'hydrogène, un atome d'halogène, un groupe hydrocarboné en C₁-C₈, linéaire ou ramifié, saturé ou insaturé, ou un groupe hydroxy.

A titre d'exemples de composés de formule (I) ou (II) ci-dessus, on peut notamment citer la 1,3-bis[3-(9,10-dioxo-9,10-dihydroanthracén-1-ylamino)propyl]urée et la 1,3-bis[3-(4-méthylamino-9,10-dioxo-9,10-dihydroanthracén-1-ylamino)propyl]urée.

Un autre objet de la présente invention est le procédé de fabrication des composés de formule (I) ci-dessus.

Il consiste à faire réagir ensemble les dérivés anthraquinoniques de formules générales (III) et (IV) suivantes 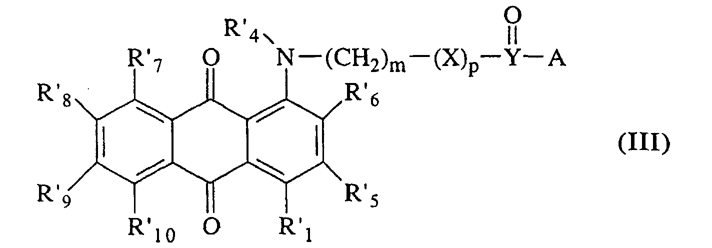 dans laquelle A représente un groupement activant comme notamment un groupe dérivé de l'imidazole ou encore un atome d'halogène, les autres symboles ayant la même signification que pour la formule (I) ; 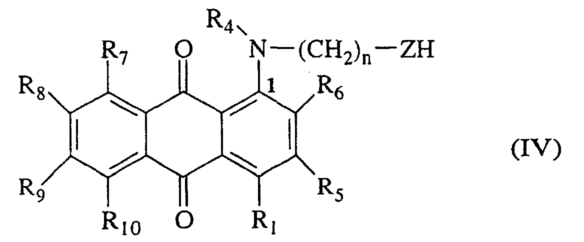 dans laquelle R₁, R₄ à R₁₀, Z et n ont la même signification que pour la formule (I), en présence d'un solvant et éventuellement d'une base, à une température de 0 à 160 °C pendant 30 minutes à 20 heures.

Les solvants convenant dans la présente invention sont de préférence des solvants dipolaires aprotiques tels que, par exemple, le N,N-diméthylacétamide ou le N,N-diméthylformamide.

Les bases éventuellement utilisées sont notamment choisies parmi la pyridine ou ses dérivés, la triéthylamine ou la diisopropyléthylamine.

Un procédé de fabrication préféré de l'invention permet d'obtenir des composés de formule (I) ci-dessus dans laquelle R₁'=R₁, R₄'=R₄, R₅'=R₅, R₆'=R₆, R₇'=R₇, R₈'=R₈, R₉'=R₉ et R₁₀'=R₁₀, n=m, Z=X et p=1. Il permet d'obtenir entre autres les composés particulièrement préférés de formule (II) ci-dessus.

Ledit procédé consiste à faire réagir un dérivé anthraquinonique de formule générale (IV) : 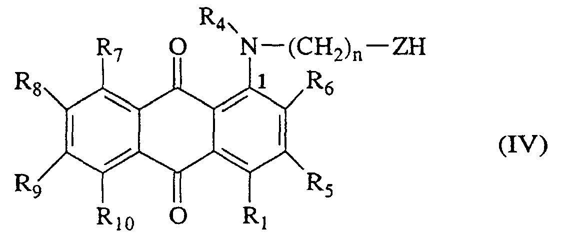 dans laquelle n, Z, R₁, R₄ à R₁₀ sont tels que définis ci-dessus pour la formule (I) ; ou un de ses sels,
avec du phosgène ou un de ses dérivés ou de ses substituts comme le carbonyldiimidazole, à une température de 0 à 160 °C pendant 30 minutes à 20 heures, en présence d'un solvant tel que défini ci-dessus, et éventuellement d'une base telle que décrite ci-dessus.

L'invention concerne également l'utilisation comme pigment d'au moins un composé de formule (I) ou (II) telle que définie ci-dessus, et de préférence de la 1,3-bis[3-(9,10-dioxo-9,10-dihydroanthracén-1-ylamino)propyl]urée et de la 1,3-bis[3-(4-méthylamino-9,10-dioxo-9,10-dihydroanthracén-1-ylamino)propyl]urée.

Cette nouvelle famille de pigments peut être utilisée dans les domaines alimentaires, cosmétiques et dans les peintures, et de préférence en cosmétique.

La composition cosmétique selon l'invention comprend, dans un milieu cosmétiquement acceptable, au moins un composé de formule (I) ou (II) telle que définie ci-dessus. Ce composé est notamment contenu en une quantité comprise entre 0,01 et 50 % en poids, de préférence entre 0,1 et 25 % en poids par rapport au poids total de la composition.

Par milieu cosmétiquement acceptable, on entend un milieu compatible avec toutes les matières kératiniques telles que la peau, les ongles, les cheveux, les cils et sourcils, les lèvres, et toute autre zone cutanée du corps et du visage.

Ledit milieu peut comprendre ou se présenter sous la forme de, par exemple, une suspension, une dispersion, une solution en milieu solvant ou hydroalcoolique, éventuellement épaissie, voire gélifiée ; une émulsion huile-dans-eau, eau-dans-huile, ou multiple ; un gel ou une mousse ; un gel émulsionné ; une dispersion de vésicules notamment lipidiques ; une lotion biphase ou multiphase ; un spray ; une poudre libre, compacte ou coulée ; ou d'une pâte anhydre.

L'homme du métier pourra choisir la forme galénique appropriée, ainsi que sa méthode de préparation, sur base de ses connaissances générales, en tenant compte d'une part de la nature des constituants utilisés, notamment de leur solubilité dans le support, et d'autre part de l'application envisagée pour la composition.

Lorsque la composition se présente sous forme aqueuse, notamment sous forme de dispersion, d'émulsion ou de solution aqueuse, elle peut comprendre une phase aqueuse qui comprend de l'eau, une eau florale et/ou une eau minérale.

Ladite phase aqueuse peut comprendre en outre des alcools tels que des monoalcools en C₁-C₆ et/ou des polyols tels que le glycérol, le butylèneglycol, l'isoprène-glycol, le propylèneglycol, le polyéthylèneglycol.

Lorsque la composition selon l'invention se présente sous la forme d'une émulsion, elle peut éventuellement comprendre en outre un tensioactif, de préférence en une quantité de 0,01 à 30 % en poids par rapport au poids total de la composition. La composition selon l'invention peut également comprendre au moins un co-émulsionnant qui peut être choisi parmi le monostéarate de sorbitane oxyéthyléné, des alcools gras tels que l'alcool stéarylique ou l'alcool cétylique, ou des esters d'acides gras et de polyols tels que le stéarate de glycéryle.

La composition selon l'invention peut encore comprendre un ou plusieurs agents épaississants dans des concentrations allant de préférence de 0 à 6 % en poids, par rapport au poids total de la composition, choisis parmi:
- les biopolymères polysaccharidiques comme la gomme de xanthane, la gomme de caroube, la gomme de guar, les alginates, les celluloses modifiées, les dérivés de l'amidon, les dérivés d'éthers de cellulose possédant des groupes ammoniums quaternaires, les polysaccharides cationiques ;
- les polymères synthétiques comme les poly(acide acrylique), la polyvinylpyrrolidone, le poly(alcool vinylique), les polymères à base de polyacrylamide ;
- le silicate de magnésium et d'aluminium.

Selon l'application envisagée, la composition peut comprendre en outre un polymère filmogène. Ceci est notamment le cas lorsque l'on souhaite préparer une composition de type vernis à ongles, mascara, eye-liner ou composition capillaire de type laque. Les polymères peuvent être dissous ou dispersés dans le milieu cosmétiquement acceptable. En particulier, le polymère peut être présent sous forme de solution dans un solvant organique ou sous forme de dispersion aqueuse de particules de polymère filmogène. Ledit polymère peut être choisi parmi la nitrocellulose, l'acétobutyrate de cellulose, les poly(butyral de vinyle), les résines alkydes, les polyesters, les polymères acryliques, les polymères vinyliques, et/ou les polyuréthannes.

La composition peut également comprendre au moins un plastifiant, qui peut être présent en une teneur allant de 1 % à 40 % en poids par rapport au poids total de la composition.

La composition selon l'invention peut également comprendre une phase grasse, notamment constituée de corps gras liquides à 25°C, tels que des huiles d'origine animale, végétale, minérale ou synthétique ; de corps gras solides à 25°C tels que des cires d'origine animale, végétale, minérale ou synthétique ; de corps gras pâteux ; de gommes ; ou de leurs mélanges.

Les compositions selon l'invention peuvent ainsi comprendre des huiles volatiles, qui s'évaporeront au contact de la peau, mais dont la présence, dans la composition cosmétique, est utile car elles facilitent l'étalement de la composition lors de l'application sur la peau. Les huiles volatiles convenant particulièrement dans l'invention présentent généralement, à 25°C, une tension de vapeur saturante au moins égale à 50 Pa (0,5 millibar).

On peut citer, par exemple, les huiles siliconées volatiles, telles que :
- les silicones volatiles cycliques ayant de 3 à 8 atomes de silicium et de préférence de 4 à 6.
- les cyclocopolymères du type diméthylsiloxane/méthylalkylsiloxane,
- les silicones volatiles linéaires ayant de 2 à 9 atomes de silicium.

On peut également citer les huiles volatiles hydrocarbonées, telles que les isoparaffines et notamment l'isododécane et des huiles fluorées.

On peut également utiliser des huiles non volatiles, parmi lesquelles on peut citer:
- les polyalkyl(C₁-C₂₀)siloxanes et notamment ceux à groupements terminaux triméthylsilyle, parmi lesquels on peut citer les polydiméthylsiloxanes linéaires et les alkylméthylpolysiloxanes tels que la cétyldiméthicone (nom CTFA) ;
- les silicones modifiées par des groupements aliphatiques et/ou aromatiques, éventuellement fluorés, ou par des groupements fonctionnels tels que des groupements hydroxyles, thiols et/ou amines ;
- les huiles de silicone phénylées, notamment celles de formule : 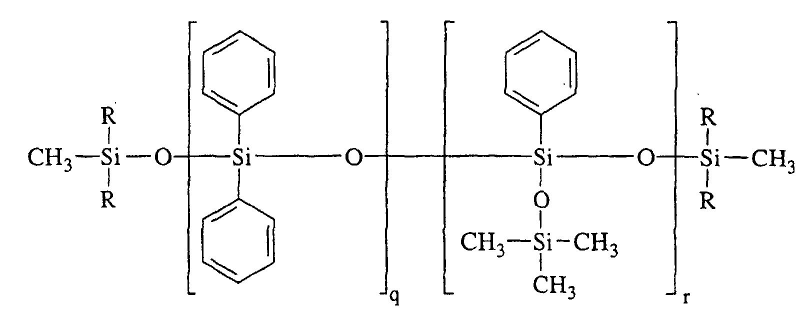
dans laquelle R représente un groupe alkyle en C₁-C₃₀, un groupe aryle ou un groupe aralkyle, q est un nombre entier compris entre 0 et 100, et r est un nombre entier compris entre 0 et 100, sous réserve que la somme est comprise entre 1 et 100 ;
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat ; les huiles de poisson, le tricaprocaprylate de glycérol, ou les huiles végétales ou animales de formule RₐCOOR_{b} dans laquelle Rₐ représente le reste d'un acide gras comportant de 7 à 19 atomes de carbone et R_{b} représente une chaîne hydrocarbonée ramifiée contenant de 3 à 20 atomes de carbone, par exemple, l'huile de Purcellin ; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales ; des esters d'acides gras ; des alcools ; des acétylglycérides ; des octanoates, décanoates ou ricinoléates d'alcools ou de polyalcools ; des triglycérides d'acides gras ; des glycérides ;
- les huiles fluorées et perfluorées.

La composition selon l'invention peut comprendre en outre d'autres corps gras, qui peuvent être choisis par l'homme du métier sur base de ses connaissances générales, de manière à conférer à à la composition finale les propriétés souhaitées, par exemple en consistance et/ou en texture. Ces corps gras additionnels peuvent être des cires, des gommes et/ou des corps gras pâteux d'origine animale, végétale, minérale ou synthétique, ainsi que leurs mélanges. On peut notamment citer les gommes de silicones ; les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés ; les cires de Candellila, d'Ouricury, de Carnauba, du Japon, le beurre de cacao, les cires de fibres de lièges ou de canne à sucre ; les huiles hydrogénées, les ozokérites, les esters gras et les glycérides ; les cires de polyéthylène et les cires obtenues par synthèse de Fischer-Tropsch ; des lanolines ; les cires de silicone ; les cires fluorées ; les polyoléfines.

La composition peut contenir en outre des nacres et/ou des charges, ainsi que d'autres pigments bien connus dans la technique.

Les nacres peuvent être présentes dans la composition à raison de 0 à 20% en poids, de préférence 8 à 15% en poids, et peuvent être choisies parmi la nacre naturelle, le mica recouvert d'oxyde de titane, d'oxyde de fer, de pigment naturel ou d'oxychlorure de bismuth ainsi que le mica titane coloré.

Les charges, qui peuvent être présentes à raison de 0 à 30% en poids, de préférence 5 à 15%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères de polymère telles que l'Expancel® (Nobel Industrie), le polytrap® (Dow Corning) et les microbilles de résine de silicone (Tospearls® de Toshiba, par exemple), le carbonate de calcium précipité, le carbonate ou l'hydrocarbonate de magnésium, des savons métalliques dérivés d'acides organiques carboxyliques ayant de 8 à 22 atomes de carbone.

La composition peut en outre comprendre un colorant hydrosoluble ou liposoluble, notamment un colorant organique naturel tel que le carmin de cochenille, et/ou un colorant de synthèse tel que les colorants halogéno-acides, azoïques, anthraquinoniques. On peut également citer des colorants minéraux tels que le sulfate de cuivre.

La composition peut comprendre en outre tout additif usuellement utilisé dans le domaine cosmétique, tel que des parfums, des huiles essentielles, des conservateurs, des actifs cosmétiques lipophiles ou hydrophiles, des hydratants, des vitamines, des acides gras essentiels, des sphingolipides, des agents autobronzants tels que la dihydroxyacétone (DHA), des filtres solaires, des agents antimousses, des agents séquestrants, des antioxydants, des agents dispersants.

Bien entendu, l'homme de l'art veillera à choisir le ou les éventuels composés complémentaires mentionnés ci-dessus, de telle manière que les propriétés avantageuses liées intrinsèquement à la composition tinctoriale selon l'invention ne soient pas, ou substantiellement pas, altérées par la ou les adjonctions envisagées.

Les compositions cosmétiques selon l'invention peuvent se présenter :
- sous la forme d'un produit de maquillage de la peau du visage, du corps ou des lèvres ou des fibres kératiniques (ongles, cils, sourcils, cheveux), tel qu'un fond de teint, une crème teintée, un fard à joues ou à paupières, une poudre libre ou compacte, un stick anti-cernes, un stick camouflant, un eye-liner, un mascara, un rouge à lèvres, un vernis-à-ongles, une composition de maquillage des cheveux ;
- sous la forme d'un produit de soin de la peau du visage ou du corps y compris le cuir chevelu, tel qu'une composition de soin (de jour, de nuit, anti-rides, hydratante, etc.) pour le visage ; une composition matifiante pour le visage ;
- sous la forme d'une composition de protection solaire ou de bronzage artificiel (autobronzant) ; ou
- sous la forme d'une composition capillaire, et notamment une crème ou un gel coiffant, une composition de teinture, d'oxydation ou directe, éventuellement sous forme de shampooing colorant.

Les exemples suivant sont destinés à illustrer l'invention et ne doivent être considérés en aucune manière comme limitant l'invention.

### Exemple 1 : préparation de la 1,3-bis[3-(9,10-dioxo-9,10-dihydroanthracén-1-ylamino)propyl]urée (formule (II) avec R₁, R₄, R₅, R₆ = H et n=3)

Dans un tricol de 250 ml, sous atmosphère d'azote, on a partiellement dissous 5 g (17,84 mmoles) de 1-[(3-aminopropyl)amino]-9,10-anthracènedione à température ambiante dans 200 ml de diméthylformamide. Puis, on a ajouté 1,5 g (0,55 éq.) de carbonyldiimidazole.

On agite d'abord le mélange 1 heure à température ambiante puis on le chauffe à 40 °C pendant 5 heures. Après filtration, lavage à l'eau et à l'acétone, puis séchage sous vide à 50 °C, on obtient 4,5 g d'une poudre rouge foncée (rendement final de 90 %).

Les caractéristiques du produit final sont les suivantes :
- point de fusion supérieur à 260 °C (mesuré sur un banc Kofler),
- spectre de masse (SSQ710, CI-DI) : m/z=587,
- analyse élémentaire (C₃₅H₃₀N₄O₅, PM=586,653) :

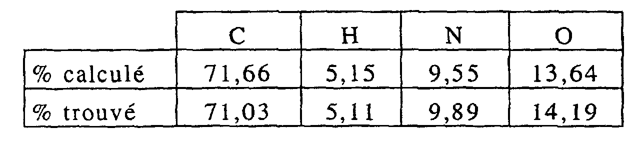

### Exemple 2 : préparation de la 1,3-bis[3-(4-méthylamino-9,10-dioxo-9,10-dihydroanthracén-l-ylamino)propyl]urée (formule (II) avec R₄, R₅ et R₆ = H, R₁=NHCH₃, et n=3)

Dans un tricol de 250 ml, sous atmosphère d'azote, on a partiellement dissous 5 g (16,27 mmoles) de 1-[(3-aminopropyl)amino]-9,10-anthracènedione à température ambiante dans 200 ml de diméthylformamide. Puis, on a ajouté 1,45 g (0,5 éq.) de carbonyldiimidazole.

On agite d'abord le mélange 1 heure à température ambiante puis on le chauffe à 40 °C pendant 6 heures. Après filtration, lavage à l'eau et à l'acétone, puis séchage sous vide à 50 °C, on obtient 3,9 g d'une poudre bleue mauve (rendement final de 73 %).

Les caractéristiques du produit final sont les suivantes :
- point de fusion supérieur à 260 °C (mesuré sur un banc Kofler),
- spectre de masse (SSQ710, EI-DEP) : m/z=644,
- analyse élémentaire (C₃₇H₃₆N₆O₅, PM=644,7280) :

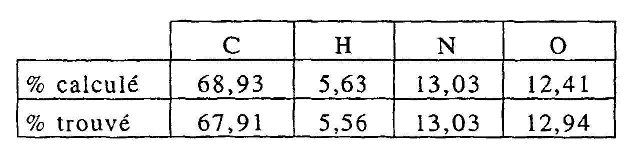

### Exemple de formulation n°1

On prépare une crème teintée de type émulsion huile-dans-eau à partir des ingrédients suivants :
- Huile de parléam 22 g
- Acide stéarique 1,5 g
- Polysorbate 60⁽¹⁾ (vendu sous la dénomination commerciale Tween® 60 par la société ICI) 0,9 g
- Alcool cétylique 0,5 g
- Mélange monostéarate de glycérol/stéarate de 2,1 g PEG 100
- Triéthanolamine 0,75 g
- Composé de l'exemple 1 5 g
- Propylèneglycol 3 g
- Cyclopentadiméthylsiloxane 3 g
- Carbopol 981 0,15 g
- Gomme de xanthane 0,2 g
- Eau qsp 100 g

### Exemple de formulation n°2

On prépare un fard à paupières à partir des ingrédients suivants :
- Talc 38 g
- Mica 20 g
- Oxychlorure de bismuth 8g
- Stéarate de zinc 3g
- Poudre de nylon 20 g
- Composé de l'exemple 2 5g
- Eau qsp 100 g

On obtient un fard à paupières bleu qui est stable et qui présente de bonnes propriétés cosmétiques.

### Exemple de formulation n°3

On prépare un rouge à lèvres à partir des ingrédients suivants :
- Cire de polyéthylène 15 g
- Composé de l'exemple 1 10 g
- Huile de parléam qsp 100 g

On obtient un rouge à lèvres rouge présentant de bonnes propriétés cosmétiques.

## Revendications

1. Composés répondant à la formule générale (I) : 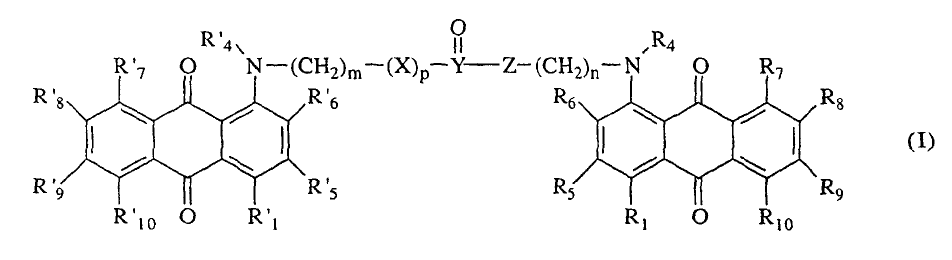 dans laquelle :
m et n, identiques ou différents, sont compris entre 1 et 20 ;
X et Z représentent chacun NH ou O ;
p vaut 0 ou 1 ;
Y représente un atome de carbone ou un atome de soufre ;
R₁, R'₁, R₅, R'₅, R₆, R'₆, R₇, R'₇, R₈, R'₈, R₉, R'₉, R₁₀, R'₁₀, identiques ou différents, représentent chacun un atome d'hydrogène, un atome d'halogène, un groupe hydrocarboné en C₁₋₈, linéaire ou ramifié, saturé ou insaturé, un groupe hydroxy,
―O-R₁₁, 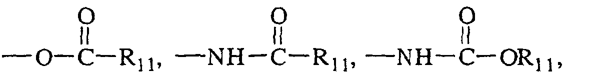 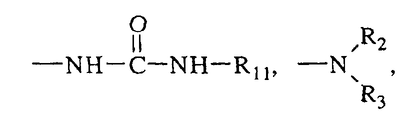 - S0₃H ou ses sels, 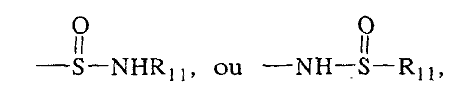
R₂, R₃, R₄, R'₄ et R₁₁, identiques ou différents, représentent chacun un atome d'hydrogène, un groupe hydrocarboné en C₁₋₈, linéaire ou ramifié, saturé ou insaturé, éventuellement hydroxylé.

2. Composés répondant à la formule générale (II) 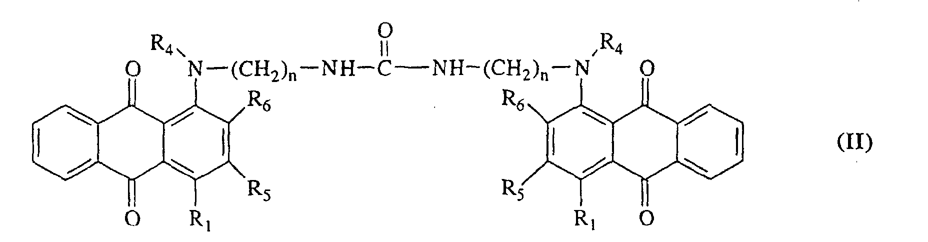 dans laquelle :
n vaut de 1 à 12,
R₁ représente un atome d'hydrogène, un atome d'halogène, un groupe hydrocarboné en C₁-C₈, linéaire ou ramifié, saturé ou insaturé, un groupe hydroxy, ou un groupe -NR₂R₃,
R₂, R₃ et R₄ représentent chacun un atome d'hydrogène ou un groupe hydrocarboné en C₁-C₈, linéaire ou ramifié, saturé ou insaturé,
R₅ et R₆ représentent chacun un atome d'hydrogène, un atome d'halogène, un groupe hydrocarboné en C₁-C₈, linéaire ou ramifié, saturé
ou insaturé, ou un groupe hydroxy.

3. Composés selon la revendication 1 ou 2, **caractérisés en ce qu'**ils sont choisis parmi la 1,3-bis[3-(9,10-dioxo-9,10-dihydroanthracén-1-ylamino)propyl]urée et la 1,3-bis[3-(4-méthylamino-9,10-dioxo-9,10-dihydroanthracén-1-ylamino)propyl]urée.

4. Procédé de fabrication des composés selon la revendication 1, **caractérisé en que l'**on fait réagir ensemble les dérivés anthraquinoniques de formules générales (III) et (IV) : 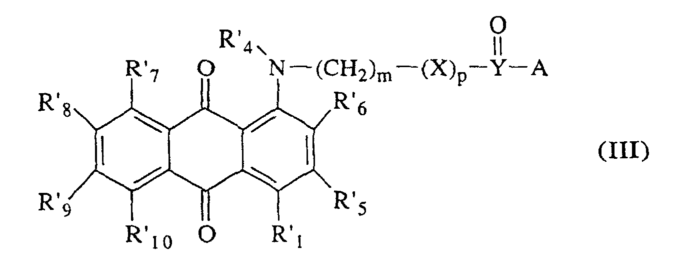 dans laquelle :
A représente un groupement activant tel qu'un groupe dérivé de l'imidazole, ou encore un atome d'halogène,
m est compris entre 1 et 20 ;
X représente NH ou O ;
p vaut 0 ou 1 ;
Y représente un atome de carbone ou un atome de soufre ;
R'₁, R'₅, R'₆, R'₇, R'₈, R'₉, et R'₁₀, identiques ou différents, représentent chacun un atome d'hydrogène, un atome d'halogène, un groupe hydrocarboné en C₁₋₈, linéaire ou ramifié, saturé ou insaturé, un groupe hydroxy,
―O―R₁₁, 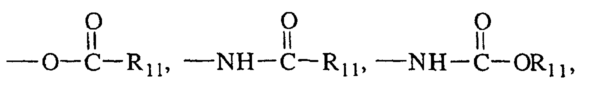 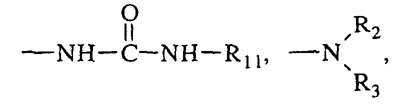 -SO₃H ou ses sels, 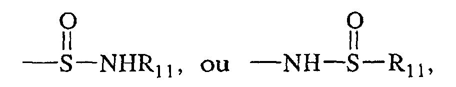
R₂, R₃, R'₄ et R₁₁, identiques ou différents, représentent chacun un atome d'hydrogène, un groupe hydrocarboné en C₁₋₈, linéaire ou ramifié, saturé
ou insaturé, éventuellement hydroxylé ; et 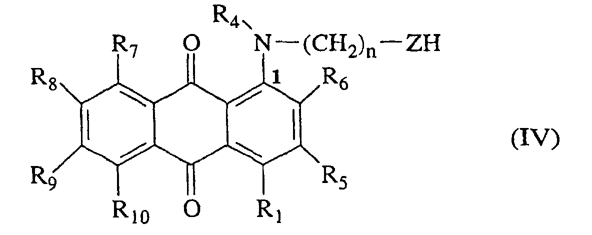
dans laquelle :
n est compris entre 1 et 20 ;
Z représente NH ou O ;
R₁, R₅, R₆, R₇, R₈, R₉, et R₁₀, identiques ou différents, représentent chacun un atome d'hydrogène, un atome d'halogène, un groupe hydrocarboné en C₁₋₈, linéaire ou ramifié, saturé ou insaturé, un groupe hydroxy,
―O―R₁₁, 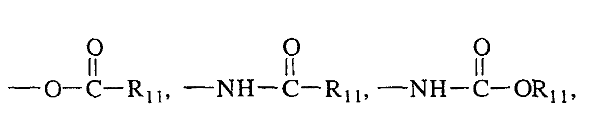 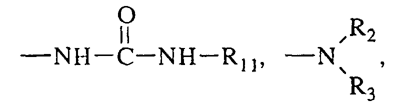 -SO₃H ou ses sels, 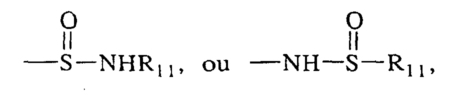
R₂, R₃, R₄ et R₁₁, identiques ou différents, représentent chacun un atome d'hydrogène, un groupe hydrocarboné en C₁₋₈, linéaire ou ramifié, saturé ou insaturé, éventuellement hydroxylé ; et
en présence d'un solvant et éventuellement d'une base, à une température de 0 à 160 °C pendant 30 minutes à environ 20 heures.

5. Procédé de fabrication **caractérisé en qu'**il consiste à faire réagir un dérivé anthraquinonique de formule générale (IV) : 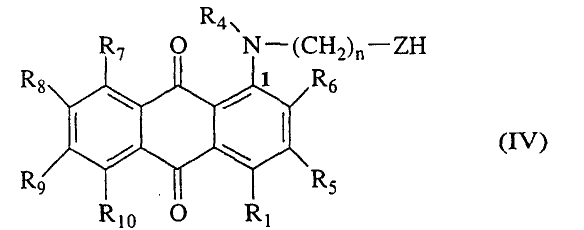 dans laquelle :
n est compris entre 1 et 20 ;
Z représente NH ou O ;
R₁, R₅, R₆, R₇, R₈, R₉, et R₁₀, identiques ou différents, représentent chacun un atome d'hydrogène, un atome d'halogène, un groupe hydrocarboné en C₁₋₈, linéaire ou ramifié, saturé ou insaturé, un groupe hydroxy,
―O―R₁₁, 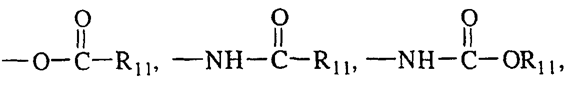 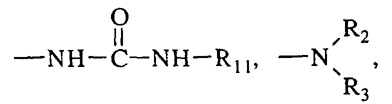 -SO₃H ou ses sels, 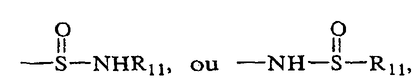
R₂, R₃, R₄ et R₁₁, identiques ou différents, représentent chacun un atome d'hydrogène, un groupe hydrocarboné en C₁₋₈, linéaire ou ramifié, saturé ou insaturé, éventuellement hydroxylé ; et
avec du phosgène ou un de ses dérivés ou de ses substituts comme le carbonyldiimidazole, à une température de 0 à environ 160 °C pendant 30 minutes à environ 20 heures, en présence d'un solvant, et éventuellement d'une base.

6. Procédé selon la revendication 4 ou 5, **caractérisé en ce que** le solvant est un solvant dipolaire aprotique.

7. Procédé selon l'une quelconque des revendications 4 à 6, **caractérisé en ce que** le solvant est le N,N-diméthylacétamide ou le N,N-diméthylformamide.

8. Procédé selon l'une quelconque des revendications 4 à 7, **caractérisé en ce que** la base est choisie parmi la pyridine ou ses dérivés, la triéthylamine ou la diisopropyléthylamine

9. Utilisation comme pigment, d'au moins un composé selon la revendication 1 ou 2.

10. Utilisation selon la revendication 9, **caractérisée en ce que** les composés sont choisis parmi la 1,3-bis[3-(9,10-dioxo-9,10-dihydroanthracén-1-ylamino)propyl]urée et la 1,3-bis[3-(4-méthylamino-9,10-dioxo-9,10-dihydroanthracén-1-ylamino)propyl]urée.

11. Utilisation comme pigment d'au moins un composé selon la revendication 1, 2 ou 3, dans une composition cosmétique.

12. Composition cosmétique, **caractérisée en ce qu'**elle comprend, dans un milieu cosmétiquement acceptable, au moins un composé selon la revendication 1 ou 2.

13. Composition cosmétique selon la revendication 12, **caractérisée en ce que** le composé est choisi parmi la 1,3-bis[3-(9,10-dioxo-9,10-dihydroanthracén-1-ylamino)propyl]urée et la 1,3-bis[3-(4-méthylamino-9,10-dioxo-9,10-dihydroanthracén-1-ylamino)propyl]urée.

14. Composition cosmétique selon la revendication 12 ou 13, **caractérisée en ce que** ledit composé est contenu en une quantité allant de 0,01 à 50 % en poids.

15. Composition cosmétique selon la revendication 14, **caractérisée en ce que** ledit composé est contenu en une quantité allant de 0,1 à 25 % en poids.

16. Composition cosmétique selon l'une quelconque des revendications 12 à 15, **caractérisée en ce que** le milieu cosmétiquement acceptable comprend ou se présente sous la forme d'une suspension, d'une dispersion, d'une solution en milieu solvant ou hydroalcoolique, éventuellement épaissie, voire gélifiée ; d'une émulsion huile-dans-eau, eau-dans-huile, ou multiple ; d'un gel ou d'une mousse ; d'un gel émulsionné ; d'une dispersion de vésicules ; d'une lotion biphase ou multiphase ; d'un spray ; d'une poudre libre, compacte ou coulée ; ou d'une pâte anhydre.

17. Composition cosmétique selon l'une quelconque des revendications 12 à 16, **caractérisée en ce qu'**elle se présente sous la forme d'un produit de maquillage de la peau du visage, du corps ou des lèvres ou des fibres kératiniques ; d'un produit de soin de la peau du visage ou du corps ; d'une composition de protection solaire ou de bronzage artificiel ; ou d'une composition capillaire.

## Patentansprüche

1. Verbindungen der allgemeinen Formel (I) 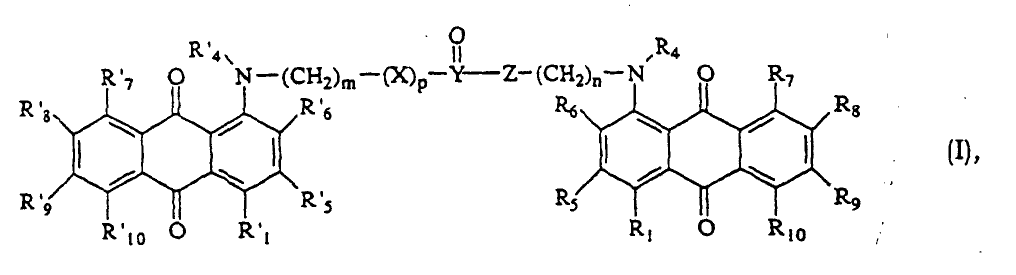 in der bedeuten:
m und n Zahlen, die gleich oder verschieden sind und im Bereich von 1 bis 20 liegen;
X und Z jeweils einen Rest NH oder 0;
p die Zahl 0 oder 1,
Y ein Kohlenstoffatom oder ein Schwefelatom;
R₁, R'₁, R₅, R'₅, R₆, R'₆, R₇, R'₇, R₈, R'₈, R₉, R'₉, R₁₀ und R'₁₀, die
gleich oder verschieden sind, jeweils ein Wasserstoffatom, ein Halogenatom, eine geradkettige oder verzweigte, gesättigte oder ungesättigte C₁-C₈-Kohlenwasserstoffgruppe, eine Hydroxygruppe,
―O―R₁₁, 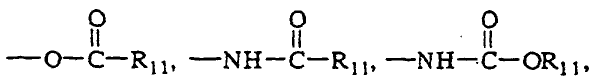 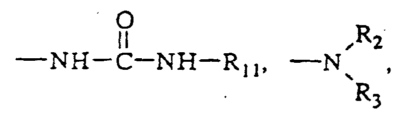 -SO₃H oder eines ihrer Salze, 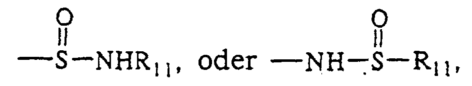
R₂, R₃, R₄, R'₄ und R₁₁, die gleich oder verschieden sind, jeweils ein Wasserstoffatom, eine geradkettige oder verzweigte, gesättigte oder ungesättigte C₁-C₈-Kohlenwasserstoffgruppe, die gegebenenfalls hydroxyliert ist.

2. Verbindungen der allgemeinen Formel (II) 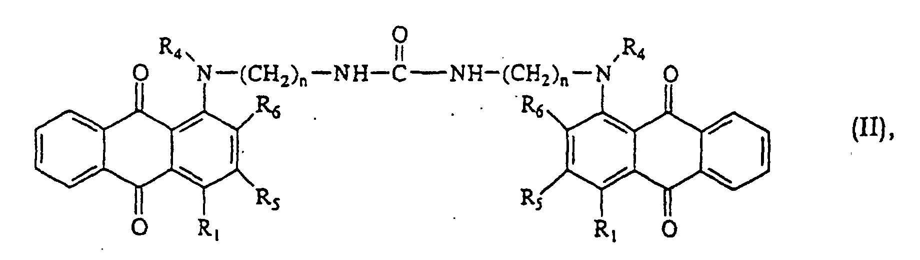 in der bedeuten:
n eine Zahl im Bereich von 1 bis 12,
R₁ ein Wasserstoffatom, ein Halogenatom, eine geradkettige oder verzweigte, gesättigte oder ungesättigte C₁-C₈-Kohlenwasserstoffgruppe, eine Hydroxygruppe oder eine Gruppe -NR₂R₃,
R₂, R₃ und R₄ jeweils ein Wasserstoffatom oder eine geradkettige oder verzweigte, gesättigte oder ungesättigte C₁-C₈-Kohlenwasserstoffgruppe,
R₅ und R₆ jeweils ein Wasserstoffatom, ein Halogenatom, eine geradkettige oder verzweigte, gesättigte oder ungesättigte C₁-C₈-Kohlenwasserstoffgruppe oder eine Hydroxygruppe.

3. Verbindungen nach Anspruch 1 oder 2, **dadurch gekennzeichnet, daß** sie unter 1,3-Bis[3-(9,10-dioxo-9,10-dihydroanthracen-1-yl-amino)-propyl]harnstoff und 1,3-Bis[3-(4-methylamino-9,10-dioxo-9,10-dihydroanthracen-1-ylamino)-propyl]harnstoff ausgewählt sind.

4. Verfahren zur Herstellung der Verbindungen nach Anspruch 1, **dadurch gekennzeichnet, daß** ein Anthrachinon-Derivat der allgemeinen Formel (III) 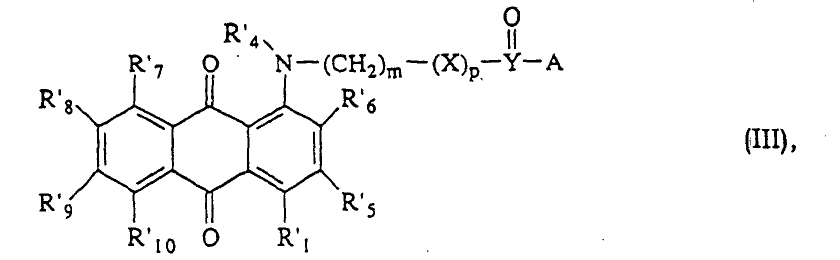 in der bedeuten:
A eine aktivierende Gruppe, wie eine Gruppe, die von Imidazol abgeleitet ist, oder ein Halogenatom,
m eine Zahl im Bereich von 1 bis 20;
X den Rest NH oder O;
p die Zahl 0 oder 1;
Y ein Kohlenstoffatom oder ein Schwefelatom;
R'₁, R'₅, R'₆, R'₇, R'₈, R'₉ und R'₁₀, die gleich oder verschieden sind, jeweils ein Wasserstoffatom, ein Halogenatom, eine geradkettige oder verzweigte, gesättigte oder ungesättigte C₁-C₈-Kohlenwasserstoffgruppe, eine Hydroxygruppe,
―O―R₁₁, 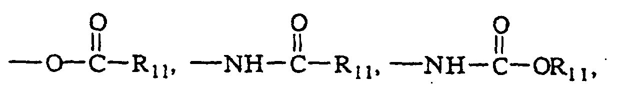 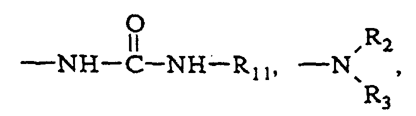 -SO₃H oder eines ihrer Salze, 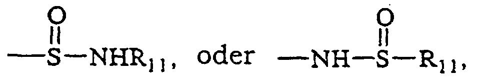
R₂, R₃, R'₄ und R₁₁, gleich oder verschieden voneinander, jeweils ein Wasserstoffatom, eine geradkettige oder verzweigte, gesättigte oder ungesättigte C₁-C₈-Kohlenwasserstoffgruppe, die gegebenenfalls hydroxyliert ist,
mit einem Anthrachinon-Derivat der Formel IV 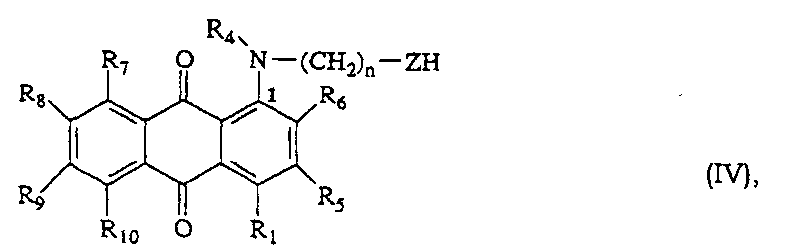 in der bedeuten:
n eine Zahl im Bereich von 1 bis 20;
Z einen Rest NH oder 0;
R₁, R₅, R₆, R₇, R₈, R₉ und R₁₀, die gleich oder verschieden sind, jeweils ein Wasserstoffatom, ein Halogenatom, eine geradkettige oder verzweigte, gesättigte oder ungesättigte C₁-C₈-Kohlenwasserstoffgruppe, eine Hydroxygruppe,
―O―R₁₁, 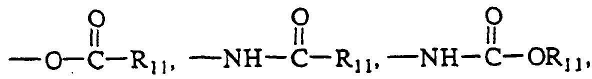 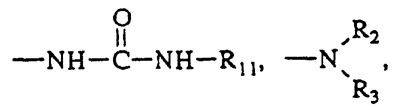 -SO₃H oder eines ihrer Salze, 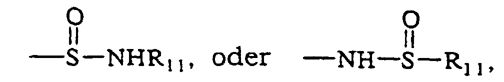
R₂, R₃, R₄ und R₁₁, die gleich oder verschieden sind, jeweils ein Wasserstoffatom, eine geradkettige oder verzweigte, gesättigte oder ungesättigte C₁-C₈-Kohlenwasserstoffgruppe, die gegebenenfalls hydroxyliert ist,
in Gegenwart eines Lösemittels und gegebenenfalls einer Base bei einer Temperatur von 0 bis 160 °C während eines Zeitraums von 30 min bis 20 h umgesetzt wird.

5. Herstellungsverfahren, **dadurch gekennzeichnet, daß** es darin besteht, ein Anthrachinon-Derivat der allgemeinen Formel (IV) 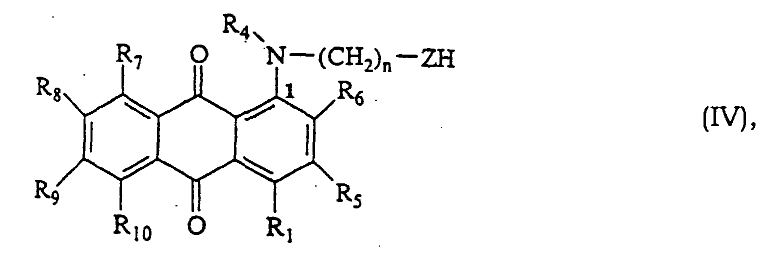 in der bedeuten:
n eine Zahl im Bereich von 1 bis 20;
Z einen Rest NH oder 0;
R₁, R₅, R₆, R₇, R₈, R₉ und R₁₀, die gleich oder verschieden sind, jeweils ein Wasserstoffatom, ein Halogenatom, eine geradkettige oder verzweigte, gesättigte oder ungesättigte C₁-C₈-Kohlenwasserstoffgruppe, eine Hydroxygruppe,
―O―R₁₁, 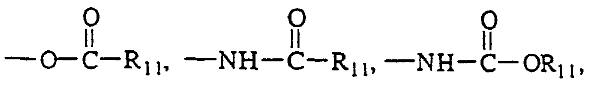 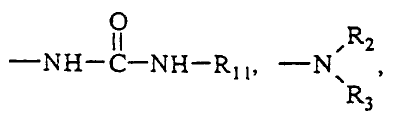 -SO₃H oder eines ihrer Salze, 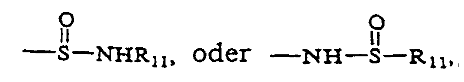
R₂, R₃, R₄ und R₁₁, die gleich oder verschieden sind, jeweils ein Wasserstoffatom, eine geradkettige oder verzweigte, gesättigte oder ungesättigte C₁-C₈-Kohlenwasserstoffgruppe, die gegebenenfalls hydroxyliert ist,
mit Phosgen oder einem seiner Derivate oder einem seiner Substitutionspodukte, wie Carbonyldiimidazol, bei einer Temperatur von 0 bis 160 °C während eines Zeitraums von 30 min bis 20 h in Gegenwart eines Lösemittels und gegebenenfalls einer Base umzusetzen.

6. Verfahren nach Anspruch 4 oder 5, **dadurch gekennzeichnet, daß** es sich bei dem Lösemittel um ein aprotisches dipolares Lösemittel handelt.

7. Verfahren nach einem der Ansprüche 4 bis 6, **dadurch gekennzeichnet, daß** es sich bei dem Lösemittel um N,N-Dimethylacetamid oder N,N-Dimethylformamid handelt.

8. Verfahren nach einem der Ansprüche 4 bis 7, **dadurch gekennzeichnet, daß** die Base unter Pyridin und seinen Derivaten, Triethylamin und Diisopropylethylamin ausgewählt wird.

9. Verwendung mindestens einer Verbindung nach Anspruch 1 oder 2 als Pigment.

10. Verwendung nach Anspruch 9, **dadurch gekennzeichnet, daß** die Verbindungen unter 1,3-Bis[3-(9,10-dioxo-9,10-dihydroanthracen-1-ylamino)-propyl]harnstoff und 1,3-Bis[3-(4-methylamino-9,10-dioxo-9,1 0-dihydroanthracen-1-ylamino)-propyl]harnstoff ausgewählt werden.

11. Verwendung mindestens einer Verbindung nach Anspruch 1, 2 oder 3 als Pigment in einer kosmetischen Zusammensetzung.

12. Kosmetische Zusammensetzung, **dadurch gekennzeichnet, daß** sie mindestens eine Verbindung nach Anspruch 1 oder 2 in einem kosmetisch akzeptablen Medium enthält.

13. Kosmetische Zusammensetzung nach Anspruch 12, **dadurch gekennzeichnet, daß** die Verbindung unter 1,3-Bis[3-(9,10-dioxo-9,10-dihydroanthracen-1-ylamino)-propyl]harnstoff und 1,3 -Bis[3-(4-methylamino-9,1 0-dioxo-9,10-dihydroanthracen-1-ylamino)-propyl]-harnstoff ausgewählt ist.

14. Kosmetische Zusammensetzung nach Anspruch 12 oder 13, **dadurch gekennzeichnet, daß** die Verbindung in einer Menge im Bereich von 0,01 bis 50 Gew.-% enthalten ist.

15. Kosmetische Zusammensetzung nach Anspruch 14, **dadurch gekennzeichnet, daß** die Verbindung in einer Menge im Bereich von 0,1 bis 25 Gew.-% enthalten ist.

16. Kosmetische Zusammensetzung nach einem der Ansprüche 12 bis 15, **dadurch gekennzeichnet, daß** das kosmetisch akzeptable Medium in Form einer Suspension, einer Dispersion, einer Lösung in einem Lösemittelmedium oder einem wäßrig-alkoholischen Medium, die gegebenenfalls verdickt oder sogar in ein Gel übergeführt ist; einer Öl-in-Wasser-Emulsion, einerWasser-in-Öl-Emulsion oder einer multiplen Emulsion, eines Gels oder eines Schaumes, eines emulgierten Gels, einer Vesikeldispersion, einer zweiphasigen Lotion oder Multiphasen-Lotion; eines Sprays; eines losen, kompaktierten oder gegossenen Pulvers; oder einer wasserfreien Paste vorliegt oder eine solche Form umfaßt.

17. Kosmetische Zusammensetzung nach einem der Ansprüche 12 bis 16, **dadurch gekennzeichnet, daß** sie in Form eines Produkts zum Schminken der Haut des Gesichts, des Körpers oder der Lippen oder der Keratinfasern; in Form eines Produkts zur Pflege der Haut des Gesichts oder des Körpers; in Form einer Sonnenschutzzusammensetzung oder einer Zusammensetzung zur künstlichen Bräunung; oder in Form einer Haarbehandlungszusammensetzung vorliegt.

## Claims

1. Compounds corresponding to the general formula (I): 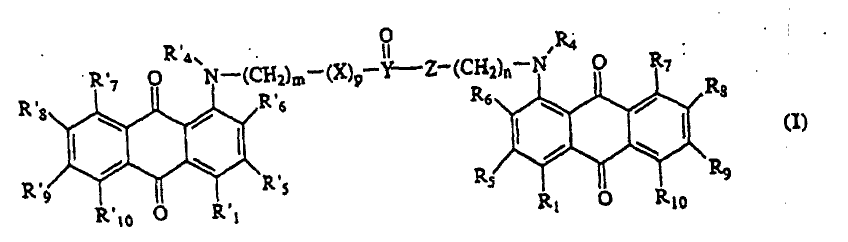 in which:
m and n, which may be identical or different, are between 1 and 20;
X and Z each represent NH or O;
p is 0 or 1;
Y represents a carbon atom or a sulphur atom;
R₁, R'₁, R₅, R'₅, R₆, R'₆, R₇, R'₇, R₈, R'₈, R₉, R'₉, R₁₀
and R'₁₀, which may be identical or different, each represent a hydrogen atom, a halogen atom, a linear or branched, saturated or unsaturated C₁₋₈ hydrocarbon-based group, a hydroxyl group,
―O―R₁₁, 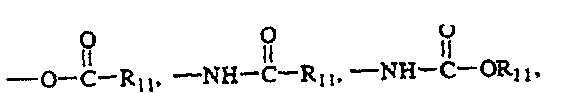 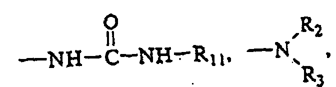 -SO₃H or salts thereof, 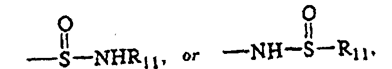
R₂, R₃, R₄, R'₄ and R₁₁, which may be identical; or different, each represent a hydrogen atom, a linear or branched, saturated or unsaturated, optionally hydroxylated, C₁₋₈ hydrocarbon-based group.

2. Compounds corresponding to the general formula (II): 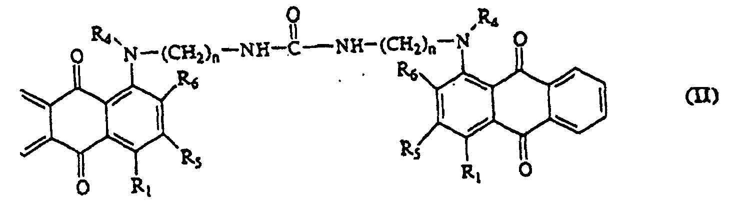 in which:
n is from 1 to 12,
R₁ represents a hydrogen atom, a halogen atom, a linear or branched, saturated or unsaturated C₁-C₈ hydrocarbon-based group, a hydroxyl group or a group -NR₂R₃,
R₂, R₃ and R₄ each represent a hydrogen atom or a linear or branched, saturated or unsaturated C₁-C₈ hydrocarbon-based group,
R₅ and R₆ each represent a hydrogen atom, a halogen atom, a linear or branched, saturated or unsaturated C₁-C₈ hydrocarbon-based group or a hydroxyl group.

3. Compounds according to Claim 1 or 2, **characterized in that** they are chosen from 1,3-bis[3-(9,10-dioxo-9,10-dihydroanthracen-1-ylamino)propyl]urea and 1,3-bis[3-(4-methylamino-9,10-dioxo-9,10-dihydroanthracen-1-ylamino)propyl]urea.

4. Process for manufacturing the compounds according to Claim 1, **characterized in that** the anthraquinone derivatives of general formulae (III) and (IV): 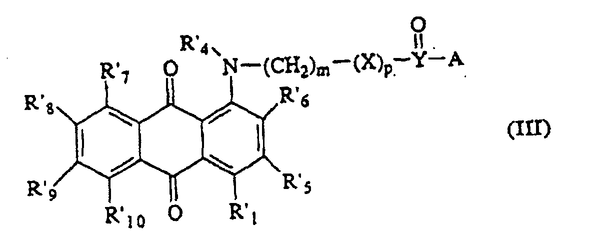 in which:
A represents an activating group such as a group derived from imidazole or a halogen atom,
m is between 1 and 20;
X represents NH or O;
p is 0 or 1;
Y represents a carbon atom or a sulphur atom;
R'₁, R'₅, R'₆, R'₇, R'₈, H'₉ and R'₁₀, which may be identical or different, each represent a hydrogen atom, a halogen atom, a linear or branched, saturated or unsaturated C₁₋₈ hydrocarbon-based group, a hydroxyl group,
―O―R₁₁, 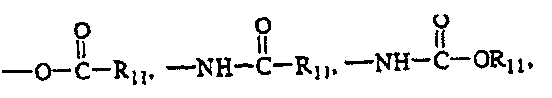 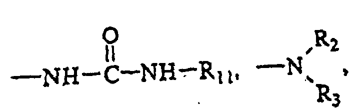 -SO₃H or salts thereof, 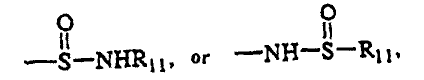
R₂, R₃, R'₄ and R₁₁, which may be identical or different, each represent a hydrogen atom, a linear or branched, saturated or unsaturated, optionally hydroxylated C₁₋₈ hydrocarbon-based group; and 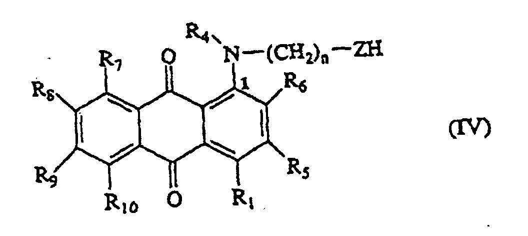
in which:
n is between 1 and 20;
Z represents NH or O;
R₁, R₅, R₆, R₇, R₈, R₉ and R₁₀, which may be identical or different, each represent a hydrogen atom, a halogen atom, a linear or branched, saturated or unsaturated C₁₋₈ hydrocarbon-based group, a hydroxyl group,
―O―R₁₁. 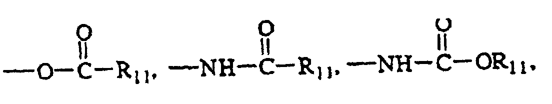 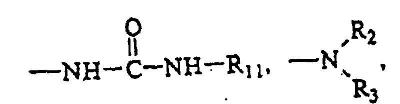 -SO₃H or salts thereof, 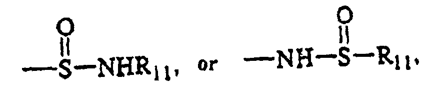
R₂, R₃, R₄ and R₁₁, which may be identical; or different, each represent a hydrogen atom, a linear or branched, saturated or unsaturated, optionally hydroxylated, C₁₋₈ hydrocarbon-based group;
are reacted together in the presence of a solvent and optionally of a base, at a temperature from 0 to 160°C for 30 minutes to 20 hours.

5. Manufacturing process, **characterized in that** it consists in reacting an anthraquinone derivative of general formula (IV): 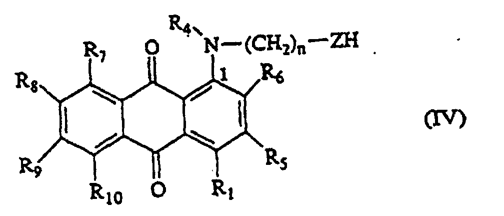 in which:
n is between 1 and 20;
Z represents NH or O;
R₁, R₅, R₆, R₇, R₈, R₉ and R₁₀, which may be identical or different, each represent a hydrogen atom, a halogen atom, a linear or branched, saturated or unsaturated C₁₋₈ hydrocarbon-based group, a hydroxyl group,
―O―R₁₁, 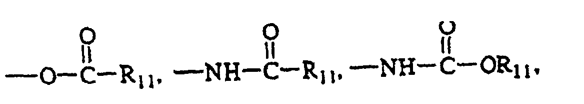 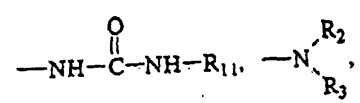 -SO₃H or salts thereof, 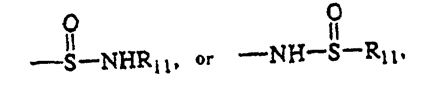
R₂, R₃, R₄ and R₁₁, which may be identical or different, each represent a hydrogen atom, a linear or branched, saturated or unsaturated, optionally hydroxylated C₁₋₈ hydrocarbon-based group;
with phosgene or one of its derivatives or of its substitutes, for instance carbonyldiimidazole, at a temperature from 0 to 160°C for 30 minutes to 20 hours, in the presence of a solvent and optionally a base.

6. Process according to Claim 4 or 5, **characterized in that** the solvent is a dipolar aprotic solvent.

7. Process according to any one of Claims 4 to 6, **characterized in that** the solvent is N,N-dimethylacetamide or N,N-dimethylformamide.

8. Process according to any one of Claims 4 to 7, **characterized in that** the base is chosen from pyridine or its derivatives, triethylamine and diisopropylethylamine.

9. Use, as a pigment, of at least one compound according to Claim 1 or 2.

10. Use according to Claim 9, **characterized in that** the compounds are chosen from 1,3-bis[3-(9,10-dioxo-9, 10-dihydroanthracen-1-ylamino) propyl] urea and 1,3-bis[3-(4-methylamino-9,10-dioxo-9,10-dihydroanthracen-1-ylamino)propyl]urea.

11. Use, as a pigment, of at least one compound according to Claim 1, 2 or 3, in a cosmetic composition.

12. Cosmetic composition, **characterized in that** it comprises, in a cosmetically acceptable medium, at least one compound according to Claim 1 or 2.

13. Cosmetic composition according to Claim 12, **characterized in that** the compound is chosen from 1,3-bis[3-(9,10-dioxo-9,10-dihydroanthracen-1-ylamino)-propyl]urea and 1,3-bis[3-(4-methylamino-9,10-dioxo-9,10-dihydroanthracen-1-ylamino)propyl] urea.

14. Cosmetic composition according to Claim 12 or 13, **characterized in that** the said compound is contained in an amount ranging from 0.01% to 50% by weight.

15. Cosmetic composition according to Claim 14, **characterized in that** the said compound is contained in an amount ranging from 0.1% to 25% by weight.

16. Cosmetic composition according to any one of Claims 12 to 15, **characterized in that** the cosmetically acceptable medium comprises or is in the form of a suspension, a dispersion, a solution in solvent or aqueous-alcoholic medium, optionally thickened, or even gelled; an oil-in-water, water-in-oil or multiple emulsion; a gel or a mousse; an emulsified gel; a dispersion of vesicles; a two-phase or multiphase lotion; a spray; a free, compact or cast powder; or an anhydrous paste.

17. Cosmetic composition according to any one of Claims 12 to 16, **characterized in that** it is in the form of a makeup product for facial or body skin or the lips or keratin fibres; a skincare product for the face or the body; an antisun composition or artificial tanning composition; or a haircare composition.
